# EUROPEAN PATENT SPECIFICATION

(11) **EP 2 096 176 B1**
(45) Date of publication and mention of the grant of the patent: **12.04.2017**
(21) Application number: 07830289.0
(22) Date of filing: 22.10.2007
(51) Int. Cl.: C12P 13/10, C12P 13/08, C12P 13/24

(54) **METHOD OF OBTAINING CRYSTAL OF HYDROCHLORIDE OF BASIC AMINO ACID**
VERFAHREN ZUR GEWINNUNG VON KRISTALLEN DES HYDROCHLORIDS EINER BASISCHEN AMINOSÄURE
PROCÉDÉ D'OBTENTION DE CRISTAL DE CHLORHYDRATE D'ACIDE AMINÉ BASIQUE

(30) Priority: 25.12.2006 JP 2006347650
(43) Date of publication of application: 02.09.2009
(73) Proprietor: Ajinomoto Co., Inc., Chuo-ku, Tokyo 104-8315 (JP)
(72) Inventor: KISHINO, Mitsuhiro, Saga-shi Saga 840-2193 (JP); KAMEI, Toshimichi, Kawasaki-shi Kanagawa 210-8681 (JP)
(74) Representative: Nicholls, Kathryn Margaret
(86) International application number: PCT/JP2007/070555
(87) International publication number: WO 2008/078448

(56) References cited:
- EP-A1- 0 534 865
- WO-A1-95/14002
- JP-A- 48 027 476
- US-A- 3 871 960
- US-A- 4 556 463

## Description

### Field of the Invention

The present invention relates to a method for obtaining crystals of a basic amino acid hydrochloride from a basic amino acid fermentation broth or an enzyme reaction solution which enzyme reaction has been catalyzed with viable cells of a basic amino acid-producing microorganism, wherein the broth or the solution contains sulfate ions therein.

### Background Art of the Invention

Usually, basic amino acid fermentation broths and enzyme reaction solutions contain sulfate ions which have been derived from the ammonium sulfate used as a nitrogen source in fermentation media and substrate solutions for the enzyme reaction, respectively.

Conventionally, in order to obtain the crystals of a basic amino acid hydrochloride from such a basic amino acid fermentation broth or an enzyme reaction solution, which broth or solution contains sulfate ions, at a high purity, first, the basic amino acid fermentation broth or the enzyme reaction solution containing sulfate ions is passed through an ammonium-type cation exchange resin column, whereby the basic amino acid is adsorbed on the resin, while the sulfate ions are removed outside from the system in the form of an ammonium sulfate solution together with the exchanged or desorbed ammonium ions. Thereafter, the ion exchange resin on which the basic amino acid has been adsorbed is washed to elute the basic amino acid with an ammonia solution and the basic amino acid is then concentrated in itself (as the free form) in the eluate. Finally, the free basic amino acid obtained is neutralized with hydrochloric acid, whereby basic amino acid hydrochloride crystals are formed and obtained from the mother liquor.

However, this method is problematic in the following respects. Namely, (1) the ammonium sulfate solution eluted must be concentrated with the use of an enormous amount of vapor to recycle the ammonium sulfate as a byproduct, and (2) a large amount of waste water will be discharged when the resin is washed.

Other methods for obtaining a basic amino acid include, for example, a method wherein a lysine fermentation broth is supplied with a potassium hydroxide solution to crystallize the lysine base (free form) (Patent document No. 1) and a method wherein a lysine fermentation broth is filtered through activated carbon to remove the cells of the lysine-producing microorganism used, the resulting filtrate being supplied with a calcium hydroxide solution to precipitate and remove the resulting calcium sulfate, and then the remainder being concentrated to remove the ammonia, whereby lysine base is obtained (Patent document No. 2). Then, the lysine base is supplied with hydrochloric acid, whereby lysine hydrochloride crystals are obtained.

However, the methods described above wherein an expensive metal hydroxide and hydrochloric acid are used as auxiliary materials and the resulting by-product metal sulfate is very cheap compared with the metal per se used, result in manufacturing flows wherein the costs for auxiliary materials are high. Furthermore, when the relevant solution after being supplied with a metal hydroxide is concentrated to a pre-determined lysine concentration, the concentrating is carried out by heat-concentrating at a high pH region, which will induce degradation of the lysine.

Moreover, these methods involve a suspension which contains metal sulfate crystals and a basic amino acid. The basic amino acid is, however, in the form of the base (free form) in the suspension, and therefore, the suspension is viscous and it is difficult to separate the metal sulfate crystals from the basic amino acid, so that a large amount of the amino acid adheres to the metal sulfate crystals to be discharged, resulting in a recovery rate of the amino acid of interest being lowered.

On the other hand, the method of the present invention involves a suspension which contains the amino acid as the hydrochloride thereof and thus is low in viscosity to allow easy separation of the metal sulfate crystals from the amino acid moiety, resulting in a recovery rate of the amino acid being raised.

In addition, there are the following problems: the aqueous solution of a metal hydroxide usually is added at a concentration of not more than 50%, and thus decreases an amino acid concentration in the system, resulting in an increased vaporing cost; meanwhile, 100% of the powder of a metal hydroxide may be added in the system, but to do so requires dangerous and difficult operations; and in addition, a step for adding metal hydroxide and a step for adding hydrochloric acid, which are required both, will complicate the production operation.

Hitherto, a metal chloride has not been reacted with a basic amino acid sulfate in a basic amino acid fermentation broth because it was believed that no anion exchange reaction occurs between the basic amino acid sulfate and the metal chloride. Namely, it was thought to be inconceivable that an equilibrium reaction would occur between the salts because the system is a solid-liquid system of the basic amino acid sulfate in the solution phase and the metal chloride in the solid phase. It was believed that, in such a solid-liquid system, even if the metal chloride is added as a solid, the metal chloride would remain as a solid, while the dissolved basic amino acid sulfate would maintained in a dissolved condition.
[Patent document No. 1]
   European Patent Publication No. 0534865.
[Patent document No. 2]
   Russian Patent Publication No. 183581.

Incidentally, according to the present invention, basic amino acids are not limited as long as they are generated by fermentation or by enzymatic methods using microbial cells as a catalyst. These amino acids include, for example, arginine, histidine and lysine. The form of the amino acids is not limited, but an L-form is preferred.

The microbes according to the present invention refer to those which are able to produce the target amino acid or those which are able to catalyze the reaction for producing the target amino acid from substrates. The former are used in fermentation methods while the latter are used in enzymatic methods. As microbes, any bacteria, yeasts, filamentous bacteria and the like may be used, bacteria being preferred. Further, bacteria may either be Gram-negative or Gram-positive. Further, a microbe may be used alone or in combination with one or more other microbes.

As known L-lysine producing bacteria and methods for breeding the same, there may be mentioned, for example, WO 95/23864, WO 96/17930, WO 2005/010175, Japanese Patent Application Laid-Open (Kokai) Sho No.56-18596, U.S. Patent No. 4346170, and Japanese Patent Application Laid-Open (Kokai) No. 2000-189180. Further, as known L-arginine producing bacteria and methods for breeding the same, there may be mentioned, for example, United States Patent Application Publication No. 2002/058315A1, Russian Patent Application No. 2001112869, EP 1170358A1 and EP 1170361A1. Moreover, as known L-histidine producing bacteria and methods for breeding the same, there may be mentioned, for example, Russian Patent Nos. 2003677 and 2119536, U.S. Patent Nos. 4,388,405, 6,344,347 and 6,258,554, Russian Patent Nos. 2003677 and 2119536, Japanese Patent Application Laid-Open (Kokai) Sho No. 56-005099 and EP1016710A. The same is the case with known L-ornithine producing bacteria and methods for breeding the same.

### DISCLOSURE OF THE INVENTION

### Problems to be Solved by the Invention

Considering the aforementioned Background Art of the Invention, it is an object of the present invention to provide a method for separating and obtaining a basic amino acid hydrochloride from a basic amino acid fermentation broth or an enzyme reaction solution which enzyme reaction has been catalyzed by viable cells of a basic amino acid-producing microbe, each of both the solution containing sulfate ions, by which method product yields and qualities are almost the same and are secured more easily as compared with the conventional technique (hereinafter, for the purposes herein, unless otherwise indicated or unless a technically different interpretation is required, the description relating to the fermentation broth shall be also applicable to that for the enzyme reaction solution).

### (Means for solving the problems)

The present inventors have strenuously studied how to achieve the above object and found that the crystals of a basic amino acid hydrochloride can be obtained in a high yield and quality by a method wherein a basic amino acid fermentation broth containing sulfate ions is supplied with a metal chloride to precipitate the sulfate ions as metal sulfate crystals, which crystals are then separated to obtain a solution, followed by cooling the solution to precipitate the basic amino acid as its hydrochloride crystals, and completed the present invention based on these findings.

Accordingly, the present invention comprises the following aspects:
[1] A method for obtaining the crystals of a basic amino acid hydrochloride comprising generating a basic amino acid using microbial cells, by fermentation in a fermentation broth, or by an enzymatic method in an enzyme reaction solution using the cells as a catalyst, wherein the broth or the solution contains sulfate ions therein, which method further comprises the steps of:
   (1) adding a solid metal chloride selected from the group consisting of calcium chloride, potassium chloride, magnesium chloride and barium chloride to the basic amino acid fermentation broth containing the basic amino acid and sulfate ions, or to the enzyme reaction solution containing the basic amino acid and sulfate ions, thereby precipitating crystals of the resulting metal sulfate,
   (2) removing the metal sulfate crystals from the basic amino acid fermentation broth or the enzyme reaction solution,
   (3) cooling the basic amino acid fermentation broth or the enzyme reaction solution from which the metal sulfate crystals have been removed while keeping the concentration of the metal sulfate below its saturated concentration,
   (4) precipitating the basic amino acid in the form of hydrochloride crystals,
   (5) separating the basic amino acid hydrochloride crystals, and
   (6) collecting them.
[2] The method according to the aspect [1], wherein the microbial cells contained in the basic amino acid fermentation broth or the enzyme reaction solution are removed before the step (1) or after the step (2).
[3] The method according to the aspect [1] or [2], wherein the basic amino acid is selected from the group consisting of arginine, lysine, ornithine and histidine.
[4] The method according to any of the aspects [1] to [3], wherein the basic amino acid fermentation broth or the enzyme reaction solution containing sulfate ions has a sulfate ion / basic amino acid equivalent ratio of 50 to 150%.
[5] The method according to any of the aspects [1] to [4], wherein the metal chloride is added in an equivalent ratio of 80 to 120% relative to the sulfate ion.

### (Effects of Invention)

A basic amino acid hydrochloride can be obtained in a good yield and quality from a basic amino acid fermentation broth containing sulfate ions by an extremely simple method according to the present invention.

### BEST MODE FOR CARRYING OUT THE INVENTION

The present invention will be described in detail below.

When the crystals of a basic amino acid hydrochloride are to be obtained according to the method of the present invention, from a basic amino acid fermentation broth containing sulfate ions, first, the chloride of a metal selected from the group consisting of calcium, potassium, magnesium and barium is added to the fermentation broth to precipitate the sulfate ions as the crystals of the resulting metal sulfate.

In this case, a basic amino acid fermentation broth containing sulfate ions for use in the present invention should be as follows. Namely, the broth should of course necessarily contain sulfate ions, and in the basic amino acid fermentation broth, the concentration of a sulfate ion is in an equivalent ratio of 50 to 150%, and preferably 90 to 110% relative to the basic amino acid. This is because if said equivalent ratio is over 150%, the amount of chloride ions to be reacted must be unnecessarily increased while, if said equivalent ratio is less than 50%, the basic amino acid may be decomposed disadvantageously because of a pH of beyond 8.5.

Here, the basic amino acid can be arginine, lysine, ornithine, histidine or a derivative thereof, and further it may either be in an optically active form, i.e. , the L- or D-form, or the racemic form thereof.

In the fermentation broth or the enzyme reaction solution of a basic amino acid for use in the present invention, the primary counterions to the contained basic amino acid are sulfate ions. Further, a fermentation broth may contain crystals of the basic amino acid produced by fermentation. In such a case, the broth must be subjected to the method of the present invention after the crystals have been removed or dissolved.

The conditions under which a metal chloride is added to precipitate the metal sulfate crystals are as follows. The metal chloride for use in the present invention can be selected as appropriate from the group consisting of calcium, potassium, magnesium and barium chlorides (comprising hydrates thereof). Of these, calcium and potassium chlorides are preferred, and of these two, potassium chloride is more preferred because it has a byproduct which can be used as a fertilizer.

The amount of the metal chloride to be added depends on the amount of the existing sulfate ions, and is an equivalent ratio of 80 to 120%, preferably 90 to 100% relative to the sulfate ions.

The microbial cells contained in the basic amino acid fermentation broth may either be removed beforehand or not. However, it is preferable that the microbial cells be removed beforehand to improve the separability of the resulting metal sulfate crystals.

Further, the basic amino acid fermentation broth may either be concentrated or not before the metal chloride is added. If the broth is concentrated, the metal chloride is preferably added before the broth is concentrated from the viewpoint of operation. This is because, in a case where the low-solubility metal sulfate crystals are to be precipitated, a large supersaturation causes an extremely high slurry concentration, whereby the crystals are difficult to separate. The most preferred method is a method wherein the broth is supplied with a metal chloride, the resulting metal sulfate crystals are separated from the remainder, and then the mother liquor is concentrated.

Potassium chloride may be added either before or after the broth is concentrated. However, in a case where calcium chloride is employed, the operations get a little bit complicated, because of repetition of crystallization/ separation, i.e., because the broth is first supplied with calcium chloride, the resulting calcium sulfate crystals are separated, the mother liquor is concentrated, and the newly resulting calcium sulfate crystals are separated.

A basic amino acid fermentation broth is preferably supplied with a metal chloride at a pH of 3 to 8.5 and at a temperature of 20 to 90°C. Specifically, the broth is preferably supplied with calcium chloride at a pH of 3 to 8.5 and at a temperature of 20 to 90°C. The broth is preferably supplied with a metal chloride other than calcium chloride at a pH of 3 to 8.5 and at a temperature of 50 to 90°C. This is because the broth which has a pH of beyond 8.5 facilitates degradation of the basic amino acid to decrease the recovery rate, while the broth which has a pH of lower than 3 increases the solubility of the basic amino acid, resulting in a decreased recovery rate. In this case, an alkali is added thereto to suppress the solubility, resulting in an increased cost. When a metal chloride is used in the above defined amount, the broth is usually in an above-mentioned range of pH, but the broth, if necessary, may be supplied with an acid or an alkali to adjust the pH appropriately. Further, the broth, which has been supplied with a metal chloride other than calcium chloride at a temperature of lower than 50°C, can not be concentrated to prevent the basic amino acid from being precipitated, making it difficult at the later process stage to get a high crystallization rate. Meanwhile, a temperature of beyond 90°C degrades the amino acid.

It is to be further noted upon the crystallization of a metal sulfate that, in the case of a solid-liquid anion exchange system wherein metal chloride crystals are present therein, enough reaction time such as two hours or more, preferably ten hours or more, should be allowed after the metal chloride has been added. In the case of a complete solution system, only the usual time due to the concentration is needed for the metal sulfate to be concentrated and crystallized.

Next, the metal sulfate crystals are removed from the basic amino acid fermentation broth. As a removing method, various centrifuges are available, and are not limited in particular. An SDC (Superdecanter-type separator) is preferred because it is easy to maintain the temperature of the mother liquor.

Subsequently, the basic amino acid fermentation broth which the metal sulfate crystals are removed from is cooled to crystallize the basic amino acid hydrochloride, while keeping the concentration of the metal sulfate below the saturation solubility of the metal sulfate. Now, before or after the fermentation broth is cooled to crystallize the basic amino acid hydrochloride, the fermentation broth may be concentrated, if necessary.

The concentration of the metal sulfate in the basic amino acid fermentation broth is necessarily kept to be below the saturation solubility of the metal sulfate, while the fermentation broth is cooled to crystallize the basic amino acid hydrochloride. For this purpose, it is important that the saturation solubility of the metal sulfate in the basic amino acid fermentation broth be acquired in advance. For example, potassium sulfate and calcium sulfate have their respective saturation solubilities of 10 g/dl and 0.05 g/dl at pH 5.5 and at 20°C in a solution of any one of arginine, lysine, ornithine and histidine. A person skilled in the art can easily determine an operation condition suitable for a given case by a preliminary test. The present invention controls concentrating to crystallize or cooling to crystallize so that the solution cannot reach the saturation solubility of the metal sulfate.

Then, the precipitated crystals of the basic amino acid hydrochloride are separated. The precipitated crystals are separated in a manner similar to that in the above-described method wherein the metal sulfate crystals are removed from the basic amino acid fermentation broth , except that an excellent in separating centrifugation apparatus, such as a basket-type centrifuge, or the like, is appropriately used. This is because, different from the above-described case where the methal sulfate crystals are separated from the basic amino acid fermentation broth, it is more important than temperature-controlling that the apparatus be used to clear the adherent mother liquor as much as possible, whereby the product purity of the basic amino acid is improved.

The crystals of the basic amino acid hydrochloride thus obtained are comparable in yield and quality to those obtained by conventional methods. Specifically, when potassium chloride is added to the arginine fermentation broth a recovery rate of as low as less than 50% is given on the first cycle But when the mother liquor which contains impurities is the starting broth any then concentrated and partly transferred for the next cycle of the fermentation broth from which the microbial cells have been separated, to reuse such that the yield is maintained, a recovery of the product with a final purity of 99% in a final yield of 90% is obtained. When calcium chloride is added to an arginine fermentation and treated similarly, a final purity of 95% in a final yield of 90% is obtained. Further, when potassium chloride is added to a lysine fermentation broth and treated a purity of 99% in a yield of 90% is obtained. When calcium chloride is added to a lysine fermentation and treated similarly, a purity of 99% in a yield of 90% is obtained.

### Examples

The present invention will be described in detail below with reference to Examples and Comparative examples, but is not limited to these Examples.

### Example 1: Production of Lysine Hydrochloride Crystals (KCl used)

The production of a lysine fermentation broth containing sulfate ions (lysine sulfate fermentation broth) : A carbon source, a nitrogen source and trace nutrients are dissolved in water, thermally sterilized and then poured into a fermenter. A suspension of a previously proliferated lysine-producing micro-organism is added thereto to start culturing. The culturing is controlled with cold water to keep a fermentation temperature of 35 to 40°C, while supplying air into the fermenter to control the level of the dissolved oxygen. The broth can be produced by a well known method, wherein the culturing is continued for about 25 to 40 hours until the production rate of the lysine is lowered in the culture medium, while this medium is being supplemented with a carbon source, a nitrogen source and a certain amount of nutrients when they become insufficient during culturing.

The lysine sulfate fermentation broth which was obtained by the above-described method was cleared of the microbial cells to give a microbial cell-free solution having the following composition.

**Table 1**

| | | | | |
|---|---|---|---|---|
| Volume | 2,384 | ml | 2,549 | g |
| Lysine base | 10.9 | w/w% | 279 | g |
| SO₄ | 4.1 | w/w% | 105 | g |
| K | 0 | w/w% | 1 | g |
| Cl | 0.1 | w/w% | 3 | g |
| pH | 7.0 | | | |

This microbial cells-free solution was concentrated in vacuum (50 mmHg) to a 37 w/w% concentration in terms of lysine base (free form lysine), supplied with 142 g of potassium chloride (KCl), and stirred for 3 hours at 60°C. Then, the suspension was, with a table separator, separated into 135 g of wet crystals of the potassium sulfate (K₂SO₄) and 729 g of lysine hydrochloride solution. This solution was supplied with 258 g of water, while keeing a temperature of 60°C to prevent the potassium sulfate from being precipitated, and then cooled in a 20°C bath over a period of 6 hours down to 20°C. The solution, when cooled to 40°C in the course of cooling, was supplied with 10 g of lysine hydrochloride dihydrate crystals as seed crystals to induce crystallization. The cooled solution was centrifuged with a table-centrifuge to give 76 g of lysine hydrochloride dihydrate crystals, which was then dried for 30 minutes at 110°C with a fluidized bed dryer to obtain 61 g of lysine hydrochloride anhydride crystals having a purity of 99%, the mother liquor being in an amount of 770 g.

On the other hand, further lysine hydrochloride anhydride crystals were obtained by a method wherein the mother liquor from a lysine fermentation broth is recycled as follows. 760 g of that final mother liquor was mixed with 1, 947 g of a microbial cells-free fermentation solution containing 202 g of lysine (in terms of lysine hydrochloride) for the next cycle, supplied with 116 g of potassium chloride, and then concentrated to have a lysine concentration of 130 g/100 g-H₂O. The suspension resulting from concentration was separated at a kept temperature of 60°C into 137 g of potassium sulfate and 890 g of a supernatant. The supernatant was supplied with 160 g of water, and cooled down from 60°C to 20°C to obtain 215 g of wet crystals of lysine hydrochloride dihydrate, which was then dried at 110°C for 30 minutes to obtain 180 g of lysine hydrochloride anhydride crystals having a purity of 99%.

This cycle was repeated nine times to obtain lysine hydrochloride anhydride crystals having a purity of 99% at a recovery rate of 90%.

### Example 2 : Production of Lysine Hydrochloride Crystals (CaCl₂ used)

A lysine sulfate fermentation broth was produced in the same way as in Example 1. Sulfuric acid was used to adjust the pH of the fermentation broth to 3.0 for storage. The microbial cells in the lysine sulfate fermentation broth were eliminated with an MF membrane to obtain a microbial cell-free solution having the following composition.

**Table 2**

| | | | | |
|---|---|---|---|---|
| Volume | 1,500 | ml | 1,628 | g |
| Lysine base | 10.7 | w/w% | 175 | g |
| SO₄ | 5.5 | w/w% | 82 | g |
| K | 0 | w/w% | 0 | g |
| Cl | 0.1 | w/w% | 0 | g |
| pH | 3.0 | | | |

This microbial cell-free solution was supplied with 12 g of calcium hydroxide (Ca(OH)₂) to neutralize it to a pH of 5.5, and stirred at 25°C for 30 minutes to precipitate calcium sulfate crystals. The resulting suspension was filtered with a Nutsche funnel to obtain 9.8 g of calcium sulfate crystals. The filtrate was supplied with 114 g of calcium chloride (CaCl₂), whereby calcium sulfate was precipitated. The suspension was filtered by the Nutsche funnel to obtain 231 g of wet crystals of calcium sulfate dihydrate and 1,475 g of a lysine hydrochloride solution. The solution was concentrated in a vacuum to 370 g, whereby a 60°C aqueous concentrate of lysine hydrochloride (concentration: 35 g/dl) was obtained. This concentrate was cooled down to 20°C to obtain a suspension of lysine hydrochloride. Then, the suspension was, with a table separator, separated, but it was not preferable that a large amount of wet crystals of calcium sulfate dihydrate be included in the suspension. Consequently, the concentrate is filtered by the Nutsche funnel to obtain wet crystals of calcium sulfate and a lysine hydrochloride solution. The lysine hydrochloride solution is cooled to 20°C to obtain a suspension of lysine hydrochloride. Then the suspension is separated with a table separator and thus 203 g of wet crystals of lysine hydrochloride dihydrate is obtained. Further, the wet crystals are dried at 110°C for 30 minutes with a fluid dryer to obtain 156 g of lysine hydrochloride anhydride crystals having a purity of 99%.

Further, by a method wherein the lysine fermentation mother liquor is recycled, the crystals of lysine hydrochloride anhydride are obtained in the same way as in Example 1. This cycle is repeated nine times to obtain crystals of lysine hydrochloride anhydride having a purity of 99% at a recovery rate of 90%.

### Example 3: Production of Arginine Hydrochloride Crystals (KCl used)

The production of an arginine fermentation broth (arginine sulfate-containing fermentation broth) containing sulfate ions: An arginine sulfate fermentation broth can be produced in the same manner as in Example 1, except that an arginine-producing microorganism is used instead of the lysine-producing microorganism.

The arginine sulfate fermentation broth which was obtained by the abobe-described method was cleared of the microbial cells to obtain a microbial cell-free solution having the following composition.

**Table 3**

| | | | | |
|---|---|---|---|---|
| Volume | | | 228 | g |
| Arginine | 44 | w/w% | 100 | g |
| SO₄ | 12 | w/w% | 27 | g |
| pH | 6.5 | | | |

This microbial cell-free solution was supplied with 42 g of potassium chloride to dissolve it, and then concentrated to an arginine concentration of 100 g/100 g-H₂O. The 60°C suspension was, by the table separator, separated into 40 g of wet crystals of potassium sulfate and 230 g of an arginine solution. Then, this solution was supplied with 30 g of water, cooled down to 20°C, and then the resulting suspension was, by the table separator, separated into 30 g of wet crystals of arginine hydrochloride and 226 g of an arginine solution.

Further, a method for obtaining arginine hydrochloride crystals was simulated using a method wherein the arginine fermentation mother liquor was recycled. It was calculated that the cycle was repeated nine times to obtain an arginine hydrochloride crystals having a purity of 99% at a recovery rate of 90%.

### (Industrial Applicability)

The present invention can be used in the fields of forage, cosmetic materials and pharmaceutical materials using a basic amino acid hydrochloride.

## Claims

1. A method for obtaining the crystals of a basic amino acid hydrochloride comprising generating a basic amino acid using microbial cells, by fermentation in a fermentation broth, or by an enzymatic method in an enzyme reaction solution using the cells as a catalyst, wherein the broth or the solution contains sulfate ions therein, which method further comprises the steps of:
(1) adding a solid metal chloride selected from the group consisting of calcium chloride, potassium chloride, magnesium chloride and barium chloride to the basic amino acid fermentation broth containing the basic amino acid and sulfate ions, or to the enzyme reaction solution containing the basic amino acid and sulfate ions, thereby precipitating crystals of the resulting metal sulfate,
(2) removing the metal sulfate crystals from the basic amino acid fermentation broth or the enzyme reaction solution,
(3) cooling the basic amino acid fermentation broth or the enzyme reaction solution from which the metal sulfate crystals have been removed while keeping the concentration of the metal sulfate below its saturated concentration,
(4) precipitating the basic amino acid in the form of hydrochloride crystals,
(5) separating the basic amino acid hydrochloride crystals, and
(6) collecting them.

2. The method as set forth in claim 1, wherein the microbial cells contained in the basic amino acid fermentation broth or the enzyme reaction solution are removed before the step (1) or after the step (2).

3. The method as set forth in claim 1 or 2, wherein the basic amino acid is selected from the group consisting of arginine, lysine, ornithine and histidine.

4. The method as set forth in any of Claims 1-3, wherein the basic amino acid fermentation broth or the enzyme reaction solution containing sulfate ions has a sulfate ion / basic amino acid equivalent ratio of 50 to 150%.

5. The method as set forth in any one of Claims 1-4, wherein the metal chloride is added in an equivalent ratio of 80 to 120% relative to the sulfate ion.

## Patentansprüche

1. Verfahren zum Erhalten der Kristalle eines Hydrochlorids einer basischen Aminosäure, welches das Erzeugen einer basischen Aminosäure unter Einsatz von mikrobiellen Zellen durch Fermentation in einer Fermentationsbrühe oder durch ein enzymatisches Verfahren in einer Enzymreaktionslösung unter Einsatz der Zellen als Katalysator umfasst, wobei die Brühe oder die Lösung Sulfationen enthält, wobei das Verfahren außerdem die folgenden Stufen umfasst:
(1) das Zugeben eines festen Metallchlorids, das aus der Gruppe ausgewählt ist, die aus Kalziumchlorid, Kaliumchlorid, Magnesiumchlorid und Bariumchlorid besteht, zu der Fermentationsbrühe einer basischen Aminosäure, die die basische Aminosäure und Sulfationen enthält, oder zu der Enzymreaktionslösung, die die basische Aminosäure und Sulfationen enthält, wodurch Kristalle des erhaltenen Metallsulfats ausgefällt werden,
(2) das Entfernen der Metallsulfatkristalle aus der Fermentationsbrühe einer basischen Aminosäure oder der Enzymreaktionslösung,
(3) das Abkühlen der Fermentationsbrühe einer basischen Aminosäure oder der Enzymreaktionslösung, aus der die Metallsulfatkristalle entfernt worden sind, während die Konzentration des Metallsulfats unter seiner Sättigungskonzentration gehalten wird,
(4) das Ausfällen der basischen Aminosäure in der Form von Hydrochloridkristallen,
(5) das Abtrennen der Kristalle des Hydrochlorids der basischen Aminosäure und
(6) das Gewinnen derselben.

2. Verfahren nach Anspruch 1, wobei die in der Fermentationsbrühe der basischen Aminosäure oder der Enzymreaktionslösung enthaltenen mikrobiellen Zellen vor der Stufe (1) oder nach der Stufe (2) entfernt werden.

3. Verfahren nach Anspruch 1 oder 2, wobei die basische Aminosäure aus der aus Arginin, Lysin, Ornithin und Histidin bestehenden Gruppe ausgewählt ist.

4. Verfahren nach einem der Ansprüche 1 bis 3, wobei die Fermentationsbrühe der basischen Aminosäure oder die Enzymreaktionslösung, die Sulfationen enthält, ein Sulfation/basische Aminosäure-Äquivalentverhältnis von 50 bis 150 % aufweist.

5. Verfahren nach einem der Ansprüche 1 bis 4, wobei das Metallchlorid in einer Äquivalentmenge von 80 bis 120 % in Bezug auf das Sulfation zugegeben wird.

## Revendications

1. Procédé d'obtention des cristaux d'un chlorhydrate d'acide aminé basique comprenant la génération d'un acide aminé basique au moyen de cellules microbiennes, par fermentation dans un bouillon de fermentation, ou par un procédé enzymatique dans une solution de réaction enzymatique en utilisant les cellules en tant que catalyseur, dans lequel le bouillon ou la solution contient des ions sulfate dans celui-ci, ledit procédé comprenant en outre les étapes de :
(1) ajout d'un chlorure métallique solide choisi dans le groupe constitué des chlorure de calcium, chlorure de potassium, chlorure de magnésium et chlorure de baryum au bouillon de fermentation d'acide aminé basique contenant l'acide aminé basique et les ions sulfate, ou à la solution de réaction enzymatique contenant l'acide aminé basique et les ions sulfate, de manière à précipiter les cristaux du sulfate métallique résultant,
(2) retrait des cristaux de sulfate métallique du bouillon de fermentation ou de la solution de réaction enzymatique d'acide aminé basique,
(3) refroidissement du bouillon de fermentation ou de la solution de réaction enzymatique d'acide aminé basique à partir duquel les cristaux de sulfate métallique ont été enlevés tout en maintenant la concentration du sulfate métallique au-dessous de sa concentration de saturation,
(4) précipitation de l'acide aminé basique sous la forme de cristaux de chlorhydrate,
(5) séparation des cristaux de chlorhydrate d'acide aminé basique, et
(6) collecte de ceux-ci.

2. Procédé selon la revendication 1, dans lequel les cellules microbiennes contenues dans le bouillon de fermentation d'acide aminé basique ou la solution de réaction enzymatique sont éliminées avant l'étape (1) ou après l'étape (2).

3. Procédé selon la revendication 1 ou 2, dans lequel l'acide aminé basique est choisi dans le groupe constitué de l'arginine, la lysine, l'ornithine et l'histidine.

4. Procédé selon l'une quelconque des revendications 1 à 3, dans lequel le bouillon de fermentation d'acide aminé basique ou la solution de réaction enzymatique contenant des ions sulfate présente un rapport équivalent d'ion sulfate / acide aminé basique de 50 à 150 %.

5. Procédé selon l'une quelconque des revendications 1 à 4, dans lequel le chlorure de métal est ajouté dans un rapport équivalent de 80 à 120 % par rapport à l'ion sulfate.
